# FASCICULE DE BREVET EUROPEEN

(11) **EP 1 070 505 B1**
(45) Date de publication et mention de la délivrance du brevet: **17.11.2004**
(21) Numéro de dépôt: 00401987.3
(22) Date de dépôt: 10.07.2000
(51) Int. Cl.: A61L 2/18, A61B 1/12

(54) **Solution aqueuse d'acide peracétique et de peroxyde d'hydrogène pour la désinfection d'endoscopes**
Wässrige Lösung aus Peressigsäure und Wasserstoffperoxid für die Desinfektion von Endoskopen
Aqueous solution of peracetic acid and hydrogen peroxide for the disinfection of endoscopes

(30) Priorité: 12.07.1999 FR 9909016
(43) Date de publication de la demande: 24.01.2001
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: Gamet, Jean-Claude, 71460 Saint Martin du Tartre (FR); Gouges, Yves, 75014 Paris (FR); Le Rouzic, Daniel, 95120 Ermont (FR)
(74) Mandataire: Conan, Philippe Claude

(56) Documents cités:
- EP-A- 0 370 850
- EP-A- 0 596 493
- EP-A- 0 658 309
- EP-A- 0 873 687

## Description

L'invention a pour objet un nouveau procédé de désinfection des endoscopes.

L'évolution actuelle des soins médicaux conduit à la multiplication des techniques exploratrices mettant en oeuvre des dispositifs de plus en plus petits, souvent fabriqués avec des matériaux thermosensibles. Il s'ensuit que ces dispositifs, ne peuvent pas être stérilisés à chaud et que, lorsqu'ils ne sont pas à usage unique, ils doivent être nettoyés et désinfectés chimiquement après chaque utilisation. Selon la nature du tissu, avec lequel le dispositif médical entre en contact lors de son utilisation, on établit trois niveaux de risques infectieux, auxquels correspondent des niveaux de traitement de désinfection requis pour un spectre d'activité à atteindre. Au haut niveau de risque infectieux, correspond un traitement de haut niveau qui doit être efficace contre tous les types microorganismes y compris les spores bactériennes. A titres d'exemples de matériels, qui doivent subir ce traitement de haut niveau, à défaut de pouvoir subir une stérilisation à chaud, on peut citer les instrumentations et moteurs de microchirurgie et coeliochirurgie, les pièces à mains en dentisterie, en stomatologie et en chirurgie, le matériel biofeedback en urologie et de nombreux endoscopes.

Une opération de désinfection chimique comprend généralement les étapes suivantes :
a) un pré-traitement, dont le but consiste à faciliter les étapes ultérieures, en empêchant par exemple les salissures de sécher ; c'est ainsi qu'à la fin de leur utilisation, les canaux des instruments médicaux en comportant, sont au plus tôt irrigués et/ou immergés dans une solution détergente et éventuellement bactéricide ;
b) un nettoyage, dont l'objectif est d'éliminer les salissures, qui va conjuguer l'action physico-chimique de la solution utilisée et les actions mécaniques effectuées manuellement ou par des machines ; c'est ainsi que les canaux des instruments médicaux en comportant, sont aspirés et rincés abondamment, et écouvillonnés ;
c) la désinfection chimique, impliquant la mise en contact du dispositif à désinfecter avec une solution désinfectante, peut se faire soit par un procédé manuel, soit par un procédé semi-automatique ou automatique. Un procédé manuel consiste en une immersion dans une solution désinfectante lorsque le dispositif est submersible, en l'application d'un désinfectant à l'aide d'un support préalablement humidifié avec une solution désinfectante, pour les dispositifs médicaux non submersibles ou en la pulvérisation dirigée sur le dispositif d'une solution désinfectante. Un procédé semi-automatique consiste en une pulvérisation au moyen d'un pulvérisateur électrique, permettant une désinfection de contact, par "dispersat dirigé" d'une solution désinfectante ; ce procédé est souvent utilisé pour nettoyer les surfaces. Un procédé automatique met en oeuvre soit des appareils spécifiques pour la désinfection de contenants, d'endoscopes ou d'instrumentation, soit des aérolyseurs pour la désinfection par voie aérienne des surfaces de locaux et des équipements qu'ils renferment ;
d) le rinçage final, dont l'objectif est d'éliminer tout résidu de produit, tout en évitant les re-contaminations ; ceci implique l'utilisation d'une eau adaptée à cette exigence qui, selon le niveau de risque, sera stérile ou filtrée ; et, lorsque le dispositif n'est pas immédiatement réutilisé,
e) le séchage, puis
f) Le stockage, ces deux dernières étapes devant, elles aussi, être effectuées dans des conditions évitant les re-contaminations du dispositif.

En désinfection manuelle, l'opérateur doit injecter avec une seringue dans les canaux de l'endoscope, les produits de nettoyage puis de désinfection. Ces étapes sont réalisées dans des bacs disposant d'un couvercle. Le désinfectant est généralement prêt à l'emploi et sa durée d'utilisation est fonction du nombre de trempages, soit en général d'une à deux semaines ou 40 opérations de désinfection.

Le glutaraldéhyde à 2 % dans l'eau, en désinfection manuelle et à 20 % dans l'eau en désinfection automatique, est aujourd'hui la matière active la plus utilisée en Europe. Le temps de désinfection efficace avec ce produit est, en désinfection manuelle, compris entre 20 minutes dans le cas d'un risque infectieux médian, et 1 heure dans le cas d'un risque d'infection élevé. En désinfection par machines, ce temps est compris entre 5 à 20 minutes environ. Cependant, le glutaraldéhyde est toxique, et les opérations le mettant en oeuvre, en particulier la désinfection manuelle, doivent être réalisées dans des espaces ventilés, voire sous des hottes aspirantes ; de plus il fixe les protéines et est peu efficace contre les spores. En effet, une solution aqueuse de 2 % en poids de glutaraldéhyde est inefficace à 20°C après 20 minutes, tant sur Bacillus cereus que sur Bacillus subtilis. Il ne devient efficace contre ces souches qu'après au moins 1 heure de trempage.

C'est pourquoi la demanderesse a recherché un substitut à ce produit ; elle s'est intéressée aux solutions aqueuses d'acide peracétique, qui sont connues pour leur efficacité sur l'ensemble des microorganismes, notamment sur les spores, lorsqu'elles sont à une concentration suffisante en peracide soit à partir d'environ 5000 ppm pour un abattement de 5 log et environ 2500 ppm pour un abattement de 3 log en 5 minutes. Cependant à cette concentration, l'acide peracétique devient corrosif pour certains des matériaux constitutifs du matériel médico-chirurgical et notamment pour leurs parties métalliques et il commence aussi à être légèrement irritant sur la peau. On sait aussi, que le peroxyde d'hydrogène seul n'est sporicide qu'à des concentrations minimales de 10 % à 20 % en poids.

La demande de brevet européen EP 0 873 687 divulgue une composition comprenant de 0, 05 % à 0, 1 % en poids d'acide peracétique, de 3 % à 4 % en poids de peroxyde d'hydrogène, de 2 % à 3 % en poids d'acide acétique, de 0,003 % à 0,006 % en poids d'un oxyde d'amine, de 0,001 % à 0,005 % en poids d'un alcool polyalcoxylé, de 0,1 % à 0,8 % en poids de dihydrogéno phosphate de sodium, de 0, 05 % à 0,1 % de pyrophosphate de sodium et si désiré de 0,0001 % à 0,05 % en poids d'un agent de coloration. Cette composition est utilisée pour désinfecter les endoscopes.

La demanderesse a donc cherché à mettre au point un procédé manuel de désinfection d'endoscopes, mettant en oeuvre l'acide peracétique à des doses non irritantes et non corrosives, qui permette d'obtenir en 20 minutes à 20°C, un abattement logarithmique de 3 log sur Bacillus cereus, soit une réduction de 10³ spores par cm³, ce résultat étant déterminé par l'analyse normalisée AFNOR T 72 301. Cela constitue une condition minimale requise pour envisager une exploitation commerciale de ce procédé en remplacement du glutaraldéhyde et du CIDEX™ PA, qui est une solution aqueuse désinfectante comprenant 800 ppm (soit environ 0,08 % en poids) d'acide peracétique et 1 % en poids de peroxyde d'hydrogène. En effet, la demanderesse a constaté qu'une solution aqueuse comprenant 800 ppm (soit environ 0,08 % en poids) d'acide peracétique et 1 % en poids de peroxyde d'hydrogène, après un an de stockage et 1 semaine d'utilisation était inefficace contre Bacillus cereus à 20°C après 20 minutes. Le choix de l'activité sporicide comme indice d'efficacité de l'acide peracétique est dicté par le haut niveau de désinfection exigé. C'est elle qui généralement requiert la dose la plus élevée en désinfectant, à cause de la présence d'une tunique sporale autour des bactéries augmentant leur résistance. Le choix de Bacillus cereus, comme cible d'étude parmi les souches indiquées dans la norme AFNOR T 72 301, à savoir Bacillus cereus CIP 7803, Bacillus subtilis et Clostridium sporogenes CIP 7939, comme cible de l'activité sporicide de l'acide peracétique, découle du résultat d'un test préliminaire, au cours duquel la demanderesse s'est aperçue que Bacillus cereus était plus facile à détruire que Bacillus subtilis avec du glutaraldéhyde, alors qu'au contraire Bacillus cereus était plus difficile à détruire que Bacillus subtilis avec de l'acide peracétique.

La demanderesse a trouvé que l'on atteignait le résultat requis, lorsque l'on mettait en oeuvre le procédé, objet de la présente demande de brevet.

L'invention a pour objet un procédé manuel de désinfection d'un endoscope, caractérisé en ce qu'il comprend une étape au cours de laquelle ledit matériel est mis en contact avec une solution aqueuse comprenant, dans de l'eau déminéralisée, de 0,10 % à 0,12 % en poids d'acide peracétique, de 2,5 % à 3,5 % en poids de peroxyde d'hydrogène, de 2 % à 4 % en poids d'acide acétique, de 0,001 % à 0,002 % en poids de GENAPOL™ 2908D, de 1 % à 2 % en poids de dihydrogénophosphate de sodium (12 H₂O), de 0,01 % à 0,03 % en poids de pyrophosphate de sodium et caractérisé en ce que 10 à 20 litres de ladite solution sont versés dans un bac de trempage et le matériel à désinfecter, y est complètement immergé et laissé pendant 20 minutes à 1 heure à température ambiante.

Par endoscope, on indique dans le procédé tel que défini ci-dessus, notamment les bronchoscopes, les laryngoscopes, les oesophagoscopes, les gastroscopes, les colonoscopes, les rectoscopes, les laparoscopes, les arthroscopes, les cystoscopes, les amnioscopes, les médiastinoscopes, les hystéroscopes, les coelioscopes, les sinuscopes, les cholédoscopes transpariétaux, les cholesdoscopes rétrogrades ou les urétéroscopes.

L'invention a plus particulièrement pour objet un procédé de désinfection, tel que défini précédemment, dans lequel l'étape de mise en contact dudit matériel avec ladite solution, est précédée d'au moins une étape de nettoyage.

Par étape de nettoyage, on indique une étape dont l'objectif est d'éliminer les salissures et qui va conjuguer l'action physico-chimique de la solution utilisée et des actions mécaniques effectuées manuellement. Par exemple, lorsque l'instrument médical comporte un canal, l'opérateur injecte la solution nettoyante dans le canal, puis celle-ci est aspirée puis l'instrument est rincé abondamment et écouvillonné. Cette opération peut être recommencée plusieurs fois, si nécessaire.

L'invention a plus particulièrement pour objet, un procédé désinfection tel que défini précédemment, dans lequel l'étape de nettoyage dudit matériel, est précédée d'une étape de pré-traitement.

Par étape de pré-traitement, on indique une étape dont le but consiste à faciliter les étapes ultérieures en empêchant par exemple les salissures de sécher, par exemple en irriguant immédiatement après la fin de leur utilisation, le canal de l'instrument médical en comportant, par une solution détergente et éventuellement bactéricide et/ou en l'immergeant dans ladite solution.

L'invention a plus particulièrement pour objet, un procédé de désinfection, tel que défini précédemment, dans lequel l'étape de mise en contact dudit matériel avec ladite solution est suivie d'une étape de rinçage puis, si nécessaire, d'une étape de séchage.

L'étape de rinçage final a pour objectif d'éliminer tout résidu de produit tout en évitant les re-contaminations ; ceci implique l'utilisation d'une eau adaptée à cette exigence qui, selon le niveau de risque, sera stérile ou filtrée ; le séchage est nécessaire lorsque le dispositif n'est pas immédiatement réutilisé.

Il est bien entendu que la présente invention inclut aussi le procédé de désinfection de plusieurs endoscopes à la fois.

Dans le cas du trempage manuel, la solution utilisée est une solution prête à l'emploi ; elle comprend donc les constituants dans les proportions indiquées précédemment. 10 à 20 litres de cette solution sont versés dans un bac de trempage et le matériel à désinfecter y est complètement immergé. Le procédé est mis en oeuvre à température ambiante, soit environ entre 15 et 35°C, généralement autour de 20 à 25°C, et le trempage dure de 20 minutes à 1 heure, suivant le risque infectieux.

La solution aqueuse d'acide peracétique mise en oeuvre, comprend, un ou plusieurs additifs choisis notamment parmi les agents tensioactifs non ioniques, les oxydes d'amines, les inhibiteurs de corrosion, les agents stabilisants, les acides forts, les colorants ou les parfums.

Comme agents tensioactifs non ioniques, on désigne dans la présente demande de brevet, le GENAPOL™ 2908D. Ce produit, disponible dans le commerce a la composition chimique suivante :

| **nom commercial** | **composition chimique** |
|---|---|
| GENAPOL™ 2908D | Mélange d'alcools alkoxylés en C₁₁, C₁₃, C₁₅ (6 à 7OE, 3OP) |

Comme oxyde d'amine, on désigne dans la présente demande de brevet, l'AROMOX™ MCD-W, dont le principe actif est le N-oxyde de cocodiméthylamine.

La solution aqueuse d'acide peracétique mise en oeuvre dans le procédé objet de la présente invention, contient comme inhibiteur de corrosion du dihydrogénophosphate de sodium, NaH₂PO₄ et comme agent stabilisant, du pyrophosphate de sodium.

Comme agents colorants stables au contact de l'acide peracétique, on désigne notamment ceux décrits dans la demande de brevet européen publiée sous le numéro EP 0 658 309. La solution aqueuse d'acide peracétique mise en oeuvre dans le procédé objet de la présente invention, peut contenir jusqu'à 0,03 % en poids d'un agent colorant.

Comme parfums stables au contact de l'acide peracétique, on désigne notamment ceux décrits dans la demande de brevet européen publiée sous le numéro EP 0 596 493. La solution aqueuse d'acide peracétique mise en oeuvre dans le procédé objet de la présente invention, peut contenir jusqu'à 0,03 % en poids de parfum.

Selon un dernier aspect de la présente invention, celle-ci a pour objet la composition mise en oeuvre dans le procédé tel que défini précédemment.

Elle contient éventuellement aussi de 0,01 % à 0,03 % en poids de N-oxyde de cocodiméthylamine et/ou de 0 % à 0,003 % en poids d'un agent colorant, et plus particulièrement l'ORANGE SOLEIL W200™.

### Exemple

### a) Trempage manuel

On a comparé les activités bactéricide, fongicide et sporicide d'une solution A, comprenant à t = 0, 1100 ppm d'acide peracétique et environ 3 % en poids de peroxyde d'hydrogène, avec celles d'une solution B, comprenant à t = 0, 800 ppm d'acide peracétique et environ 1 % en poids de peroxyde d'hydrogène, en mettant en oeuvre les méthodes d'analyse décrites respectivement dans les normes EN 1040 (activité bactéricide), EN 1275 (activité fongicide), AFNOR 72180 (activité virucide) et AFNOR T 72301 (activité sporicide).

On constate des activités bactéricide, et fongicide similaires pour les deux solutions après un temps de contact défini par la norme considérée ; la détermination de l'activité sporicide conduit aux résultats recensés dans le tableau suivant (temps de contact : 20 minutes ; température : 20°C ; abattement en spores/ml) :

| SOUCHES | glutaraldéhyde à 2% dans l'eau | SOLUTION A | SOLUTION B |
|---|---|---|---|
| Bacillus cereus | inefficace après 20 minutes | 10³ après 20 minutes | < 10³ après 20 minutes |
| Bacillus Subtilis | inefficace après 20 minutes | 10⁵ après 20 minutes | |

On constate donc que la solution A obéit de façon inattendue à l'exigence imposée en matière d'activité sporicide sur Bacillus cereus.

## Revendications

1. Procédé manuel de désinfection d'un endoscope, **caractérisé en ce qu'**il comprend une étape au cours de laquelle ledit matériel est mis en contact avec une solution aqueuse comprenant, dans de l'eau déminéralisée, de 0,10 % à 0,12 % en poids d'acide peracétique, de 2,5 % à 3,5 % en poids de peroxyde d'hydrogène, de 2 % à 4 % en poids d'acide acétique, de 0,001 % à 0,002 % en poids de GENAPOL™ 2908D, de 1 % à 2 % en poids de dihydrogénophosphate de sodium (12H₂O), de 0,01 % à 0,03 % en poids de pyrophosphate de sodium et **caractérisé en ce que** 10 à 20 litres de ladite solution sont versés dans un bac de trempage et le matériel à désinfecter y est complètement immergé et laissé pendant 20 minutes à 1 heure à température ambiante.

2. Procédé tel que défini à la revendication 1 précédemment, dans lequel l'étape de mise en contact dudit matériel avec ladite solution, est précédée d'au moins une étape de nettoyage.

3. Procédé tel que défini à la revendication 2, dans lequel l'étape de nettoyage dudit matériel, est précédée d'une étape de pré-traitement.

4. Procédé tel que défini à l'une des revendications 1 à 3, dans lequel l'étape de mise en contact dudit matériel avec ladite solution, est suivie d'une étape de rinçage puis, si nécessaire, d'une étape de séchage.

5. Procédé tel que défini à l'une des revendications 1 à 4, **caractérisé en ce que** la solution aqueuse d'acide peracétique mise en oeuvre, comprend jusqu'à 0,03% en poids d'un agent colorant.

6. Procédé tel que défini à l'une des revendications 1 à 5, **caractérisé en ce que** la solution aqueuse d'acide peracétique mise en oeuvre, comprend jusqu'à 0,03 % en poids d'un parfum.

7. Composition sous forme d'une solution aqueuse, dans de l'eau déminéralisée, comprenant :
de 0,10 % à 0,12 % en poids d'acide peracétique,
de 2,5 % à 3,5 % en poids de peroxyde d'hydrogène,
de 2 % à 4 % en poids d'acide acétique,
de 0,001 % à 0,002 % en poids de GENAPOL™ 2908D,
de 1 % à 2 % en poids de dihydrogénophosphate de sodium (12H₂O),
de 0,01 % à 0,03 % en poids de pyrophosphate de sodium.

8. Composition telle que définie à la revendication 7, contenant en outre de 0,01 % à 0,03 % en poids de N-oxyde de cocodiméthylamine et/ou de 0 % à 0,003 % en poids d'un agent colorant, et plus particulièrement l'ORANGE SOLEIL W200™.

9. Utilisation d'une composition telle que définie à l'une des revendications 7 ou 8, pour désinfecter un endoscope.

## Patentansprüche

1. Manuelles Verfahren zur Desinfektion eines Endoskops, **dadurch gekennzeichnet, dass** es einen Schritt umfasst, in dessen Verlauf das Material mit einer wässrigen Lösung in Kontakt gebracht wird, die, in entmineralisiertem Wasser, 0,10 Gew.-% bis 0,12 Gew.-% Peressigsäure, 2,5 Gew.-% bis 3,5 Gew.-% Wasserstoffperoxid, 2 Gew.-% bis 4 Gew.-% Essigsäure, 0,001 Gew.-% bis 0,002 Gew.-% GENAPOL™ 2908D, 1 Gew.-% bis 2 Gew.-% Natriumdihydrogenphosphat (12 H₂O), 0,01 Gew.-% bis 0,03 Gew.-% Natriumpyrophosphat umfasst, und **dadurch gekennzeichnet, dass** 10 bis 20 Liter dieser Lösung in eine Tauchwanne geschüttet werden und das zu desinfizierende Material vollständig darin eingetaucht und 20 Minuten bis 1 Stunde bei Umgebungstemperatur darin belassen wird.

2. Verfahren nach dem vorstehenden Anspruch 1, wobei dem Schritt des In-Kontakt-Bringens des Materials mit der Lösung mindestens ein Putzschritt vorausgeht.

3. Verfahren nach Anspruch 2, wobei dem Schritt des Putzens des Materials ein Vorbehandlungsschritt vorausgeht.

4. Verfahren nach einem der Ansprüche 1 bis 3, wobei auf den Schritt des In-Kontakt-Bringens des Materials mit der Lösung ein Spülschritt und dann, wenn nötig, ein Trocknungsschritt folgt.

5. Verfahren nach einem der Ansprüche 1 bis 4, **dadurch gekennzeichnet, dass** die verwendete wässrige Peressigsäurelösung bis zu 0,03 Gew.-% eines Färbemittels umfasst.

6. Verfahren nach einem der Ansprüche 1 bis 5, **dadurch gekennzeichnet, dass** die verwendete wässrige Peressigsäurelösung bis zu 0,03 Gew.-% eines Duftstoffs umfasst.

7. Zusammensetzung in Form einer wässrigen Lösung in entmineralisiertem Wasser, welche folgendes umfasst:
0,10 Gew.-% bis 0,12 Gew.-% Peressigsäure,
2,5 Gew.-% bis 3,5 Gew.-% Wasserstoffperoxid,
2 Gew.-% bis 4 Gew.-% Essigsäure,
0,001 Gew.-% bis 0,002 Gew.-% GENAPOL™ 2908D,
1 Gew.-% bis 2 Gew.-% Natriumdihydrogenphosphat (12 H₂O),
0,01 Gew.-% bis 0,03 Gew.-% Natriumpyrophosphat.

8. Zusammensetzung nach Anspruch 7, die ferner 0,01 Gew.-% bis 0,03 Gew.-% Cocodimethylamin-N-oxid und/oder 0 Gew.-% bis 0,003 Gew.-% eines Färbemittels und insbesondere ORANGE SOLEIL W200™ umfasst.

9. Verwendung einer Zusammensetzung nach einem der Ansprüche 7 oder 8 zur Desinfektion eines Endoskops.

## Claims

1. Manual method of disinfecting an endoscope, **characterized in that** it comprises a step during which the said equipment is brought into contact with an aqueous solution comprising, in demineralized water, from 0.10% to 0.12% by weight of peracetic acid, from 2.5% to 3.5% by weight of hydrogen peroxide, from 2% to 4% by weight of acetic acid, from 0.001% to 0.002% by weight of GENAPOL™ 2908D, from 1% to 2% by weight of sodium dihydrogen phosphate (12H₂O), from 0.01% to 0.03% by weight of sodium pyrophosphate and **characterized in that** 10 to 20 litres of the said solution are poured into a soaking tank and the equipment to be disinfected is completely immersed therein and left for 20 minutes to 1 hour at room temperature.

2. Method as defined in the preceding Claim 1, in which the step of bringing the said equipment into contact with the said solution is preceded by at least one cleaning step.

3. Method as defined in Claim 2, in which the step of cleaning the said equipment is preceded by a pretreatment step.

4. Method as defined in one of Claims 1 to 3, in which the step of bringing the said equipment into contact with the said solution is followed by a rinsing step and then, if necessary, a drying step.

5. Method as defined in one of Claims 1 to 4, **characterized in that** the aqueous solution of peracetic acid used comprises up to 0.03% by weight of a colouring agent.

6. Method as defined in one of Claims 1 to 5, **characterized in that** the aqueous solution of peracetic acid used comprises up to 0.03% by weight of a perfume.

7. Composition in the form of an aqueous solution, in demineralized water, comprising:
from 0.10% to 0.12% by weight of peracetic acid,
from 2.5% to 3.5% by weight of hydrogen peroxide,
from 2% to 4% by weight of acetic acid,
from 0.001% to 0.002% by weight of GENAPOL™ 2908D,
from 1% to 2% by weight of sodium dihydrogen phosphate (12H₂O),
from 0.01% to 0.03% by weight of sodium pyrophosphate.

8. Composition as defined in Claim 7, containing, in addition, from 0.01% to 0.03% by weight of cocodimethylamine N-oxide and/or from 0% to 0.003% by weight of a colouring agent, and more particularly ORANGE SOLEIL W200™.

9. Use of a composition as defined in either of Claims 7 and 8, for disinfecting an endoscope.
